Europäisches Patentamt

European Patent Office

Office européen des brevets

(11) Veröffentlichungsnummer: **0 178 506**
**B1**

(12) **EUROPÄISCHE PATENTSCHRIFT**

(45) Veröffentlichungstag der Patentschrift: 08.08.90

(51) Int. Cl.⁵: **A 61 K 6/04, C 22 C 5/06**

(21) Anmeldenummer: **85112165.7**

(22) Anmeldetag: **25.09.85**

(54) **Verwendung von Silber-Palladium-Legierungen als Werkstoff zum Aufbrennen von Dentalkeramiken.**

(30) Priorität: **19.10.84 DE 3438288**

(43) Veröffentlichungstag der Anmeldung:
**23.04.86 Patentblatt 86/17**

(45) Bekanntmachung des Hinweises auf die
Patenterteilung:
**08.08.90 Patentblatt 90/32**

(84) Benannte Vertragsstaaten:
**AT BE CH DE FR GB IT LI LU NL SE**

(56) Entgegenhaltungen:
**DE-A-3 146 794**
**DE-C-3 204 743**
**NL-A-8 001 820**
**US-A-3 929 474**
**US-A-4 350 526**

(73) Patentinhaber: **Degussa Aktiengesellschaft**
**Weissfrauenstrasse 9**
**D-6000 Frankfurt am Main 1 (DE)**

(72) Erfinder: **Schöck, Gernot, Dipl.-Ing.**
**Bahnhofstrasse 56**
**D-6451 Bruchköbel (DE)**
Erfinder: **Hausselt, Jürgen, Dr. Dipl.-Phys.**
**Weinbergring 37**
**D-6456 Langenselbold (DE)**
Erfinder: **Rothaut, Josef, Dr. Dipl.-Phys.**
**247 Harmon Avenue**
**Fort Lee, NJ 07024 (US)**
Erfinder: **Hathaway, Doris**
**Lahnstrasse 20**
**D-6450 Hanau 7 (DE)**

Courier Press, Leamington Spa, England.

# EP 0 178 506 B1

**Beschreibung**

Die Erfindung betrifft die Verwendung von Silber-Palladium-Legierungen als Werkstoff zu Aufbrennen von Dentalkeramiken.

Zur Herstellung von Kronen, Brücken und Inlays für die konservierende Zahnheilkunde werden seit langem Edelmetal-Legierungen eingesetzt. In zunehmendem Umfang werden hierfür sogenannte Aufbrennlegierungen verwendet, bei denen aus ästhetischen Gründen die metallischen Kronen bzw. Brücken ganz oder teilweise mit einer zahnfarbenen Keramik überzogen werden.

Hierfür haben sich besonders hochgoldhaltige Legierungen auf AuPtPd-Basis mit ca. 80% Gold, aber auch sogenannte goldreduzierte Legierungen auf AuPd- bzw. AuPdAg-Basis bewährt. Aufgrund der hohen Edelmetallpreise und aufgrund von Kostendämpfungsmaßnahmen im Gesundheitswesen werden in jüngster Zeit jedoch vermehrt billigere Legierungen auf Palladium-Basis als Aufbrennlegierungen eingesetzt. Diese Legierungen sind entweder goldfrei oder enthalten nur wenige Gew.% Gold. Die Nachteile dieser Legierungen liegen in ihrer unphysiologisch hohen Härte, dem hohen Schmelzintervall und ihrer unbefriedigenden Lötbarkeit, insbesondere nach der keramischen Verblendung.

Aufgrund ihres hohen Palladium-Gehaltes (über 75%) können solche Legierungen auf keinen Fall im Graphittiegel aufgeschmolzen werden.

Dies erschwert und verteuert ihre Verarbeitung im dentallabor.

Neben Legierungen auf Palladium-Basis werden auch solche auf Palladium-Silber-Basis als Aufbrennlegierungen eingesetzt. Solche Legierungen sind beispielsweise in der US—PS—4,350,526 und in der DE—PS—32 04 743 beschreiben. Trotz weitgehender Erfüllung einiger der für Aufbrennlegierungen geforderten Eigenschaften, wie Härte, Schmelzintervall und Festigkeit, besitzen diese Legierungen weiterhin auch Nachteile. Durch den noch recht hohen Pd-Gehalt von 45—70% ist die Kohlenstoffaufnahme beim Schmelzen im Graphittiegel noch erheblich und kann zur Blasenbildung in den aufgebrannten Dentalkeramiken führen. Als weiterer Nachteil ist der immer noch sehr hohe Preis dieses Legierungstyps anzusehen.

Aus der US—PS—3,929,474 ist eine Legierung auf der Basis von Silber-Palladium bekannt, die 28 bis 64% Silber, 37,5 bis 57,7% Palladium, 0,5 bis 7% Chrom, Eisen, Indium und/oder Zinn und 0 bis 5% Silizium, Nickel, Kobalt, Tantal und/oder Titan enthalten. Bei diesen Legierungen wurde Wert auf ihre Anlaufbeständigkeit gelegt. Ihre thermische Expansion liegt bei der von Porzellan. Für moderne keramische Aufbrennmassen, deren thermischer Ausdehnungskoeffizient bei Werten größer als $15,5 \cdot 10^{-6} K^{-1}$ liegt, sind sie nur bedingt brauchbar. Auch zeigen sie keine optimalen Gießeigenschaften, da sie bei höheren Temperaturen nur eine geringe Duktilität besitzen und es daher beim Abkühlen der Gußstücke zu Brüchen kommen kann.

Aus der DE—A—3146794 ist eine Legierung auf Palladium-Gold-basis mit 20 bis 85% Palladium, 0 bis 55% Gold, 0 bis 40% Silber, 1 bis 15% Zinn und/oder Indium, jeweils 0,05—1% eines oder mehrer der Elemente Iridium, Rhenium, Ruthenium, Silizium, Eisen, Kupfer und Zink, 0,1 bis 3% Gallium und je 0,05 bis 1% Tantal, Wolfram und/oder Yttrium bekannt. Auch diese Legierung zeigt im wesentlichen die bekannten Nachteile der Palladiumbasislegierungen.

Es war daher Aufgabe der vorliegenden Erfindung, Silber-Palladium-Legierungen zum Aufbrennen von Dentalkeramik zu entwickeln, die preisgünstig und leicht zu verarbeiten sind, die ein möglichst breites Anwendungsspektrum besitzen, sich auch mit Dentalkeramikmassen verblenden lassen, die hohe thermische Ausdehnungskoeffizienten aufweisen, und die beim Abkühlen nach dem Vergießen nicht zu Brüchen niegen.

Diese Aufgabe wird erfindungsgemäß durch die Verwendung von Silber-Palladium-Legierungen mit 50 bis 70% Silber, 15 bis 45% Palladium, 0,1 bis 2% Ruthenium und/oder Rhenium, 0,2 bis 10% Kobalt und/oder Kupfer, 0,2 bis 12% Indium und/oder Zinn, 0,2 bis 5% Gallium, 0,05 bis 2% Tantal und/oder Wolfram, 0 bis 5% Gold und/oder Platin und 0 bis 2% Zink gelöst.

Besonders gut eignen sich Legierungen, die 50 bis 60% Silber, 35 bis 45% Palladium, 0,1 bis 2% Ruthenium und/oder Rhenium, 0,2 bis 10% Kupfer, 0,2 bis 10% Indium und/oder Zinn, 0,2 bis 4% Gallium und 0 bis 1,5% Zink enthalten.

Diese Legierungen können so eingestellt werden, daß sie einen thermischen Ausdehnungskoeffizienten zwischen 20 bis 600°C von größer als $15,5 \cdot 10^{-6} K^{-1}$, eine Härte von mehr als 150 HV5 und Solidustemperaturen oberhalb 1075°C besitzen. Dadurch lassen sie sich mit modernen dentalkeramischen Massen, die ebenfalls einen hohen thermischen Ausdehnungskoeffizienten aufweisen, problemlos verblenden.

Die erfindungsgemäßen Legierungen zeichnen sich aus durch ein exzellentes Gieß- und Formfüllungsverhalten sowie durch eine außergewöhnliche Feinkörnigkeit. Trotz ihres niedrigen Schmelzintervalls besitzen sie eine hohe Festigkeit bei der Brenntemperatur der Keramik. Die Legierungen weisen ferner eine hohe Anlaufbeständigkeit und Korrosionsfestigkeit auf. Sie sind nicht anfällig für Kohlenstoffaufnahme und die damit verbundenen Probleme bei der Verblendung mit Dentalkeramik. Der Zusatz von Gallium führt in Verbindung mit den übrigen Legierungsbestandteilen zu einer deutlichen Verbesserung der Duktilität bei höheren Temperaturen, insbesondere bei 650°C, so daß die Gefahr von Gußbrüchen nicht mehr besteht. Außerdem verbessert Gallium die Gießeigenschaften der Silber-Palladium-Legierungen wesentlich.

Die Legierungen eignen sich aufgrund ihrer niedrigen Härte auch als Dental-Gußlegierungen, so daß der bei kombinierten Arbeiten erforderliche aufwendige, und zum Teil wenig erfolgreiche Lötvorgang entfallen kann. Andererseits weisen alle erfindungsgemäßen Legierungen eine sehr gute Lötbarkeit auf, so daß Lötungen sowohl vor als auch nach der keramischen Verblendung völlig problemlos durchgeführt werden können. Es has sich ferner gezeigt, daß die Festigkeit der Legierungen über den Tantal- bzw. Wolfram-Gehalt sowie durch den Einsatz von Kobalt gesteuert werden kann, ohne daß die Härte zu sehr ansteigt oder die Duktilität der Legierungen wesentlich abnimmt. Es ist daher möglich, erfindungsgemäße Legierungen auch für zahntechnischen Modellguß, d.h. für herausnehmbaren Zahnersatz einzusetzen.

Die folgende Tabelle zeigt die Haupteigenschaften einiger erfindungsgemäßer Legierungen.

Tabelle

| Leg. Nr. | Zusammensetzung in Massen - % | | | | | | | | | | | | Schmelz-intervall °C | Guß-härte HV5 | 0,2%-Dehn-gren-ze MPa | Bruch dehng % | Therm. Ausdehng $20°C-600°C$ $10^{-6}K^{-1}$ |
|---|---|---|---|---|---|---|---|---|---|---|---|---|---|---|---|---|---|
| | Ag | Pd | Re | Ru | Co | Cu | Ga | In | Sn | Ta | W | Sons-tige | | | | | |
| 1 | 51 | 40,5 | 0,8 | -- | -- | 2,0 | 0,5 | 3,5 | 1,5 | -- | 0,2 | | 1217-1158 | 217 | 530 | 10,5 | 16,1 |
| 2 | 51 | 40,2 | -- | 0,8 | 1,0 | -- | 1,0 | 5,0 | 0,5 | 0,5 | -- | | 1220-1130 | 235 | 560 | 7,5 | 16,1 |
| 3 | 55 | 39,2 | -- | 0,7 | -- | 1,0 | 0,5 | 2,5 | 1,0 | -- | 0,1 | | 1200-1125 | 195 | 415 | 14,0 | 16,6 |
| 4 | 55 | 39,0 | 0,6 | -- | -- | 1,2 | 1,0 | 2,0 | 0,5 | 0,2 | -- | Zn0,5 | 1190-1120 | 210 | 470 | 11,5 | 16,6 |
| 5 | 60 | 35,0 | -- | 0,5 | -- | 0,5 | 0,5 | 2,5 | 0,7 | 0,3 | -- | | 1195-1125 | 180 | 410 | 12,5 | 17,3 |
| 6 | 59 | 33,0 | 0,5 | -- | -- | 1,5 | 1,0 | 1,4 | 0,5 | 0,1 | -- | Pt3 | 1205-1120 | 195 | 430 | 12,5 | 16,9 |
| 7 | 64 | 30,4 | 0,6 | -- | 1,5 | -- | 0,5 | 1,2 | 0,8 | 0,2 | 0,3 | Au0,5 | 1164-1095 | 170 | 380 | 13,7 | 17,5 |
| 8 | 67 | 28,1 | -- | 0,8 | 1,0 | 0,9 | 0,3 | 0,3 | 1,2 | 0,2 | 0,2 | | 1158-1088 | 162 | 368 | 14,8 | 17,8 |

EP 0 178 506 B1

# EP 0 178 506 B1

**Patentansprüche**

1. Verwendung von Silber-Palladium-Legierungen, bestehend aus 50 bis 70% Silber, 15 bis 45% Palladium, 0,1 bis 2% Ruthenium oder Rhenium, 0,2 bis 10% Kupfer und/oder Kobalt, 0,2 bis 12% Indium und/oder Zinn, 0,2 bis 5% Gallium, 0,05 bis 2% Tantal und/oder Wolfram, 0 bis 5% Gold und/oder Platin und 0 bis 2% Zink, als Werkstoffe zum Aufbrennen von Dentalkeramiken.

2. Verwendung von Silber-Palladium-Legierungen, bestehend aus 50 bis 60% Silber, 35 bis 45% Palladium, 0,1 bis 2% Ruthenium und/oder Rhenium, 0,2 bis 10% Kupfer, 0,2 bis 10% Indium und/oder Zinn, 0,2 bis 4% Gallium und 0 bis 1,5% Zink, als Werkstoffe zum Aufbrennen von Dentalkeramiken.

**Revendications**

1. Utilisation d'alliages Argent/Palladium, qui consistent en 50 à 70% d'argent, 15 à 45% de Palladium, 0,1 à 2% de Ruthénium ou de Rhénium, 0,2 à 10% de Cuivre et/ou de Cobalt, 0,2 à 12% d'Indium et/ou d'Etain, 0,2 à 5% de Gallium, 0,05 à 2% de Tantale et/ou de Tungstène, 0,5 à 5% d'or et/ou de Platine, et 0 à 2% de Zinc, en tant que matériau pour la calcination de céramique dentaire.

2. Utilisation d'alliages Argent/Palladium se composant de 50 à 60% d'Argent, 35 à 45% de Palladium, 0,1 à 2% de Ruthénium et/ou de Rhénium, 0,2 à 10% de Cuivre, 0,2 à 10% d'Indium et/ou d'Etain, 0,2 à 4% de Gallium et 0 à 1,5 de Zinc en tant que matériau pour la calcination de céramiques dentaires.

**Claims**

1. The use of silver-palladium alloys consisting of 50 to 70% silver, 15 to 45% palladium, 0.1 to 2% ruthenium or rhenium, 0.2 to 10% copper and/or cobalt, 0.2 to 12% indium and/or tin, 0.2 to 5% gallium, 0.05 to 2% tantalum and/or tungsten, 0 to 5% gold and/or platinum and 0 to 2% zinc as materials for the firing-on of dental ceramics.

2. The use of silver-palladium alloys consisting of 50 to 60% silver, 35 to 45% palladium, 0.1 to 2% ruthenium and/or rhenium, 0.2 to 10% copper, 0.2 to 10% indium and/or tin, 0.2 to 4% gallium and 0 to 1.5% zinc as materials for the firing-on of dental ceramics.